# EUROPEAN PATENT APPLICATION

(11) **EP 4 759 437 A1**
(43) Date of publication of application: **17.06.2026**
(21) Application number: 24220335.4
(22) Date of filing: 16.12.2024
(51) Int. Cl.: B05C 17/005, A61M 5/178, A61M 5/31, B05C 17/01

(54) **PISTON**

(71) Applicant: medmix Switzerland AG, 9469 Haag (Rheintal) (CH)
(72) Inventor: SPOEGLER, Thomas, 8197 Rafz (CH); GOLDING, Philippa, 9422 Staad SG (CH)
(74) Representative: Manitz Finsterwald Patent- und Rechtsanwaltspartnerschaft mbB

(57) **Abstract**

The invention relates to a piston (10) having a front end (12) and a rear end (14), with an outer wall (16) connecting the front end (12) to the rear end (14), wherein the outer wall (16) comprises a sealing seat (18) configured for receipt of an O-ring (20), and wherein the sealing seat (18) has a non-uniform cross-section in plane parallel to a longitudinal axis (26) of the piston (10), and with the cross-section at any axial height of the sealing seat (18) being circular in a plane perpendicular to the longitudinal axis (26) of the piston (10).

## Description

The invention relates to a piston having a front end and a rear end, with an outer wall connecting the front end to rear end.

In state of the art plunger systems that comprise a piston to eject a (liquid) material out of said system often the same major issue arises which is the issue of dripping once the dispensing motion has been completed. That is, once the dispensing mechanism comes to rest, the (liquid) material stored inside said system that has already come near the dispensing outlet without actively being dispensed, drips out of said outlet. This happens because of the rather high pressures that are still present inside said plunger system.

In order to overcome this issue several kinds of sealing apparatuses at and around the mentioned pistons have already been considered. However, it has shown that none of them can provide good sealing qualities without negatively affecting the dispensing motion of the dispenser.

It is therefore an object of the invention to provide a piston with which the issue of dripping in plunger system can be reduced. Furthermore, it is an object of the invention to provide a piston with which friction in general can be reduced and the overall precision of the plunger movement can be improved.

All of the above objects are solved by the piston according to the invention, that is, a piston having a front end and a rear end, with an outer wall connecting the front end to the rear end, wherein the outer wall comprises a sealing seat configured for receipt of an O-ring, and wherein the sealing seat has a non-uniform cross-section in plane parallel to a longitudinal axis of the piston, with a cross-section at any axial height of the sealing seat being circular in a plane perpendicular to the longitudinal axis of the piston.

The invention introduces a piston designed to reduce dripping and friction by creating a defined suck-back effect. This is achieved through conical groove design that preloads and compresses the O-ring when an axial force is applied. The reason for this is that a sealing seat for the O-ring is provided that comprises a non-uniform cross section in a plane parallel to a longitudinal axis of the piston while simultaneously comprising a circular cross section in a plane perpendicular to said longitudinal axis at any axial height thereof.

The special design of the sealing seat allows the O-ring to be preloaded and compressed when an axial force is applied on the piston. That is, in a default position, when no axial force is applied to the piston, the preload of the O-ring is at its minimum. Then, when a force is applied, i.e. when an extrusion of material is initiated, the O-ring rolls along the longitudinal axis to a final position and is compressed. Thereby, the pressure that act along said longitudinal axis is managed.

By having the O-ring rolling along the longitudinal axis rather than sliding, i.e. rubbing, along said axis, the overall friction can be reduced drastically. This eventually also leads to a more smooth, even and linear response of the piston upon extrusion as there is no initial friction or stick-slip effect that needs to be overcome before the actual extrusion of material can begin.

Additionally, when the axial force on the piston is released, the preload on the O-ring is relieved, which leads to the piston slightly retracting back towards its default position since the O-ring is decompressed. This slight movement of the piston creates a suck-back effect since the inner volume is increased such that dripping can be reduced or even prevented when an extrusion motion is paused.

Tests have even shown that the mechanism according to the invention can reduce or even (completely) eliminate dripping and friction inside the discharging mechanism compared to existing solutions according to the state of the art. With the invention a more reliable and precise operation can further be achieved.

According to an embodiment of the invention the sealing seat comprises a continuous outer surface such that the O-ring can roll along a continuous surface rather than slipping or sliding over a discontinuous one.

According to a further embodiment the sealing seat is provided in the form of a conically shaped O-ring groove. Said conically shaped groove is provided such that its cross section that lies in a plane perpendicular to the longitudinal axis of the piston tapers from a rear end towards a front end of the piston. This allows the O-ring in particular to easily roll over the continuous surface of the sealing seat.

In this connection it is further noted that the rear end is an actuation end of the piston. That is, the axial movement of the piston follows the direction from said rear end towards a front end of the piston and vice versa.

According to a further embodiment a depth of the sealing seat is larger towards the front end of the piston than a depth of the sealing seat at the rear end of the piston. That is, since the sealing seat may be shaped conically, it can comprise a larger cross section at its rear end than at its front end. Consequently, this leads to the depth at the rear end of the sealing seat, i.e. the distance between an outer surface of the piston and the outer surface of the sealing seat, to be smaller than the depth at the front end of the sealing seat.

According to another embodiment an angle of an inner wall of the sealing seat with respect to the longitudinal axis of the piston that passes from the front end to the rear end is selected in the range of 5° to 20°. This defines how much the cross section of the sealing seat in a plane perpendicular to the longitudinal axis increases from the front end of the piston respectively the sealing seat to its rear end.

According to a further embodiment of the invention a minimum O-ring compression at a front end of the sealing seat is selected in the range of 4 to 10%, especially 5 to 9% and most preferably of 7%.

Additionally or alternatively a maximum O-ring compression at a rear end of the sealing seat is selected in the range of 10 to 20%, especially 12 to 16% and most preferably of 14%.

In this connection it is noted that the minimum and maximum compression is defined as a reduction in cord diameter. That is, when the O-ring is compressed, its cross section goes from an originally circular cross section to a rather oval cross section such that it goes from having a constant radius (respectively diameter) to having a minor and a major semi-axis with the minor semi-axis being smaller than the major semi-axis and the diameter being compressed by 4 to 10% respectively 10 to 20 % at the front end respectively the read end of the sealing seat.

According to another embodiment a cross section of the O-ring comprises a cord diameter in the range of 0.7 to 2.5 mm, especially in the range of 1 to 2.2 mm, in particular 1.1 mm to 2.1. In this connection it is noted that the cord diameter is defined as the diameter of the cross section of the O-ring itself, i.e. it defines how thick said O-ring is, and not the inner or outer diameter of the ring that is spanned by said O-ring.

Said cord diameter of the O-ring may in particular have a size of 1.2 mm, 1.78 mm or 2 mm.

The inner diameter of the O-ring can lie in the range of 5 to 100 mm, in particular 8 to 40mm. In this connection it should be noted that the inner diameter of the O-ring may be smaller than at least one outer diameter of the sealing seat along an axial height of the sealing seat, i.e. the O-ring may be stretched as it is moved from the front end to the rear end of the sealing seat.

The outer diameter of the O-ring can lie in the range of 6 to 110 mm, in particular 8 to 44 mm.

In this connection it is further noted that the O-ring according to the invention can generally be defined according ISO 3601.

According to a further embodiment of the invention a length between a front end of the sealing seat and a rear end of the sealing seat is the sum of the O-ring cord diameter in its relaxed state and an axial play of said O-ring, in particular with said axial play of the O-ring being in the range of d1*0.1 to d1 *0.5. That is, the length of the sealing seat along the longitudinal direction is larger than the diameter of the O-ring to give it an axial play that allows said O-ring to roll from the front end to the rear end and vice versa. Hence, the length of the sealing seat can be given by the sum of said diameter and said axial play.

According to a further embodiment the conically shaped O-ring groove of the sealing seat comprises a rounded inner front angle, in particular with said inner front angle comprising a radius in the range of d1*0.01 to d1*0.5. The front angle of the O-ring groove, i.e. the sealing seat, may be the transition between the conically shaped outer surface of the sealing seat and a front end face of the sealing seat onto which the O-ring may rest when being in its initial position.

Additionally or alternatively the conically shaped O-ring groove of the sealing seat may comprise a rounded inner rear angle, in particular with said inner rear angle comprising a radius in the range of d1*0.01 to d1*0.5. Said rear angle of the O-ring groove, i.e. the sealing seat, may be the transition between the conically shaped outer surface of the sealing seat and a rear end face of the sealing seat onto which the O-ring may rest when it is in its compressed state.

According to a further embodiment the outer wall of the piston extends cylindrically from the rear end to the sealing seat, in particular to the rear end of the sealing seat. That is, when looking at the piston according to the invention as a whole, its rear part, i.e. the part that extends from the rear end to the sealing seat, in particular to the rear end of the sealing seat, is shaped cylindrically with a constant diameter in its cross section.

Additionally or alternatively the outer wall of the piston may further extend conically from the sealing seat to the front end of the piston, in particular from the rear end of the sealing seat to the front end of the sealing seat. That is, again, when looking at the piston according to the invention as a whole its outer surface may be conically shaped at its front part, i.e. the part extending from the sealing seat, in particular the rear end of the sealing seat, to the front end of the piston. Hence, the overall diameter of the cross section of the piston may taper towards the front end.

According to another embodiment the sealing seat comprises a top wall and a bottom wall extending transversely to the longitudinal axis, wherein the bottom wall extends further from the longitudinal axis than the top wall. Said top and bottom wall may be configured as front respectively back faces onto which the O-ring can rest in its decompressed respectively compressed state. In this connection it is noted that the bottom wall may extend further from the longitudinal axis than the top wall. This may be caused due to the fact that the outer surface of the sealing seat being formed conically such that the bottom wall is located at a greater distance from a center of the piston, i.e. its central longitudinal axis, than the top wall. Thus, even though both walls may comprise the same length, the bottom wall may extend further from the center of the piston than the top wall. Obviously, this embodiment can also be achieved with top and bottom walls that do not comprise the same length.

This embodiment can also be achieved by providing top and bottom walls that extend in different angles with respect to the longitudinal axis of the piston such that their respective outer ends lie in different longitudinal planes even though they might comprise the same length.

According to an alternative embodiment the sealing seat comprises a top wall and a bottom wall extending transversely to the longitudinal axis, wherein the bottom wall extends the same distance from the longitudinal axis as the top wall. By providing top and bottom walls that to do not comprise the same length, said two walls may nevertheless extend to a position that lies in the same plane with respect to the longitudinal axis, i.e. the center of the piston. That is, even though the sealing seat may be shaped conically such that a starting point from the top wall may be located closer to the center of the piston than a starting point of the bottom wall, their respective end points may be positioned in the same plane parallel to the longitudinal axis.

This embodiment can also be achieved by providing top and bottom walls that extend in different angles with respect to the longitudinal axis of the piston such that their respective outer ends lie in the same longitudinal plane.

According to a further embodiment the sealing seat comprises a top wall and a bottom wall extending transversely to the longitudinal axis, wherein the top wall and the bottom wall extend parallel to one another. According to this embodiment both the top and the bottom wall may for example extend in a right angle to the longitudinal axis of the piston.

According to a further embodiment an outer end of the top wall is closer to the longitudinal axis than an outer end of the bottom wall. That is, the outer ends of the top and bottom wall may not lie in the same longitudinal plane.

According to another embodiment the sealing seat comprises an outer diameter in the range of 5 to 60 mm, with said diameter reducing from the rear end to the front end of the piston. The diameter of the sealing seat may be taken along a plane that is perpendicular to the longitudinal axis of the piston respectively the sealing seat. Thus, the diameter of the cross section of the sealing seat in said plane is larger at its rear end than at its front end.

According to another embodiment of the invention the O-ring is made out of an elastomeric material, in particular thermoset materials, such as or thermoplastic materials, such as butadiene rubber (BR), butyl rubber (IIR), chlorosulonated polyethylene (CSM), epichlorohydrin rubber (ECH, ECO), ethylene propylene diene monomer (EPDM), ethylene propylene rubber (EPR), fluoroelastomer (FKM), nitrile rubber (NBR, HNBR, HSN, Buna-N), perfluoroelastomer (FFKM), polyacrylate rubber (ACM), polychloropene (CR), polyisoprene (IR), polysulfide rubber (PSR), polytetrafluoroethylene (PTFE), sanifluor (FEPM) silicone rubber (SiR),styrene-butadiene rubber (SBR), thermoplastic polyolefin (TPO) LDPE, HDPE, LLDPE, ULDPE, thermoplastic polyurethane (TPI), polyether, polyester, thermoplastic ethereseterelastomers (TEEEs) copolyesters, thermoplastic polyamide (PEBA), meld processible rubber (MPR) or thermoplastic vulcanizate (TPV). The precise material(s) can be chosen based on their chemical compatibility, application temperature, sealing pressure, lubrication requirements, durometer, size and cost.

According to a further aspect of the invention a cartridge optionally filled with a mastic material is provided with the cartridge comprising a piston according to the invention. The piston may be provided inside the cartridge such that the piston is configured to urge the material stored inside said cartridge out of a dispensing outlet.

The cartridge according to the invention may further comprise a dispensing outlet, such as a mixing tip, as well as a dispensing mechanism configured to move the piston from a rear end of the cartridge to a front end and potentially vice versa.

The invention is further described in connection with the following Figures which show:
- Fig. 1:: a sectional view of a part of the piston according to invention in a first state;
- Fig. 2: a sectional view of a part of the piston according to the invention in a second state;
- Fig. 3A:: a sectional view of the entire piston according to the invention; and
- Fig. 3B:: a side view of the piston according to the invention.

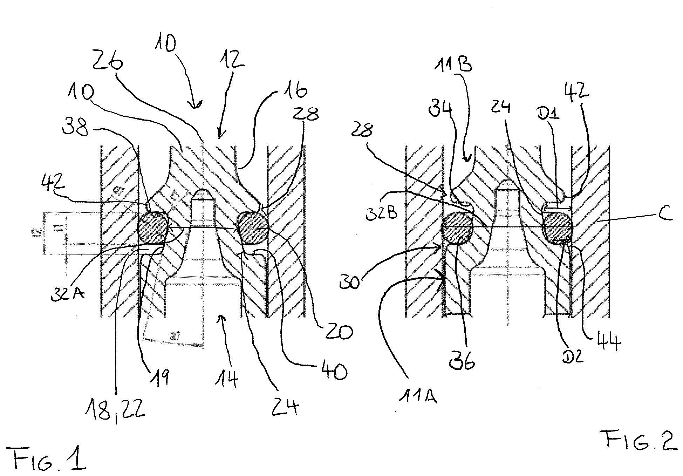

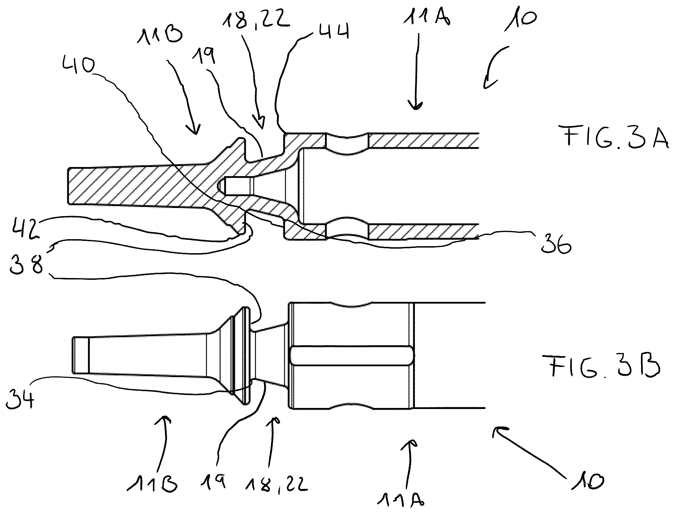

Fig. 1 shows a partial sectional view of a piston 10 according to the invention that is arranged in a cylindrical cartridge C. The piston 10 comprises a front end 12 and a rear end 14, with said rear end 14 being the actuation end of the piston 10. It can further be seen in Figs. 1 and 2 that the piston 10 can basically be divided into two parts - even though the piston 10 itself is made of one piece - which are divided by a sealing seat 18 that is configured to accommodate a sealing ring 20 (see also Figs. 3A and 3B).

The lower part 11A of the piston 10 which is located at the rear end 14, i.e. in Figs. 1 and 2 below the sealing seat 18, comprises a cylindrical outer shape, thereby having a constant outer diameter along the longitudinal axis 26 of the piston 10.

The upper part 11B of the piston 10 which is located at the front end 12, i.e. in the Figures above the sealing seat 18, comprises a generally conical outer shape with an outer diameter that decreases towards the front end 12 of the piston 10.

The sealing seat 18 is located between said lower and upper part 11A, 11B. It is configured for the receipt of a sealing ring 20 in the form of an O-ring 20. Therefore, the sealing seat 18 is provided in the form an O-ring groove 22.

Such a piston 10 (without the sealing ring 20) can also be seen in Figs. 3A and 3B.

Said groove 22 comprises a continuous outer surface 19 such that the O-ring can fit into said groove 22. Furthermore, the sealing seat 18 comprises a conical outer shape with a circular cross section at any axial height of the sealing seat 18 in a plane perpendicular to the longitudinal axis 26 of the piston 10 ('perpendicular cross section' in the following). A cross section of the sealing seat 18 that is taken along a plane parallel to the longitudinal axis 26, however, is non-uniform according to the invention.

The non-uniformity is also caused by the fact that the largest outer diameter of the perpendicular cross section of the sealing seat 18 is always smaller than the largest outer diameter of the perpendicular cross section of the lower part 11A of the piston 10. That is, when looking from the rear end 14 to the front end 12 of the piston 10 it comprises a step in its outer shape that defines the rear end 30 of the sealing seat 18. This can also be seen in Figs. 3A and 3B.

Said step is formed by a bottom wall 40 that extends from an outer end 44 towards the longitudinal axis 26 of the piston for a defined length. The bottom wall 40 terminates at a rounded rear angle 36 that forms the transition to the conically shaped outer wall 19 of the sealing seat 18. A length of the bottom wall 40 is chosen such that the O-ring 20 can lean against said bottom wall 40 when a pressure is applied to the piston 10 that is large enough to cause the O-ring 20 to move from its initial, uncompressed, position at the front end 28 of the sealing seat 18 of the piston 10 to its final, compressed position at the rear end 30 of the sealing seat 18.

The sealing seat 18 further comprises a top wall 38 that forms the front end 28 of the sealing seat 18. Said top wall 38 extends from an outer end 42 towards the longitudinal axis 26 until it reaches a rounded front angle 34 which defines the transition to the conically shaped outer wall 19 of the sealing seat 18. Accordingly, the O-ring can lean against the top wall 38 when it is in its initial, uncompressed state.

The respective outer ends 42, 44 of the top wall 38 respectively the bottom wall 40 can either lie in the same longitudinal plane or in different planes. The examples shown in Figs. 1 to 3B show an embodiment in which the outer ends 42 and 44 lie in different longitudinal planes. That is, outer end 42 is located nearer to the central longitudinal axis 26 of the piston 10 than outer end 44.

The shown embodiment can either be realized by providing top and bottom walls 38, 40 that comprise appropriately chosen lengths and/or front and rear angles 34, 36.

In the embodiments shown, the top and bottom wall 38, 40 extend parallel to one another in a 90 Degree angle with respect to the longitudinal axis 26. Furthermore, they comprise the same length such that their respective outer ends 42, 44 do not lie in the same longitudinal plane since the conical shape of outer wall 19 causes their respective starting points, i.e. front and rear angles 34, 36, to not be lying in the same longitudinal plane.

In this connection it is further noted that the front and back angles 34, 36 can comprise a radius r1 respectively r2 that each lie in the range of d1*0.01 to d1*0.5 with d1 defining the cord diameter of the cross-section of the O-ring 20 which will be discussed later on.

Because of the conical outer shape of the sealing seat 18, said perpendicular cross section further comprises a diameter that is the largest at a rear end 30 of the sealing seat 18 and the smallest at a front end 28 of the sealing seat 18. It can thus be seen in Fig. 2, for example, that a depth D1 of the sealing seat 18 is larger towards the front end 28 of the sealing seat 18 respectively the front end 12 of the piston 10 than towards the rear end 30 of the sealing seat 18 respectively the rear end 14 of the piston 10. This means that the O-ring 20 has less space to unfold at the rear end 30 of the sealing seat 18 than at the front end 28, thereby leading to a compression of the O-ring 20 when it is located at the back.

The above described conical shape of outer wall 16 can further be defined by angle a1 that is defined between the central longitudinal axis 26 of the piston 10 and the inner wall 24. In particular, said angle a1 can lie in the range of 5° to 20°.

The height, i.e. the length I2, of the sealing seat 18, which is the distance between the top wall 38 and the bottom wall 40, is defined by the cord diameter d1 of the O-ring 20 and an axial play l1. Said axial play l1 needs to be provided in order to allow the O-ring 20 to move, especially to roll, inside the sealing seat 18 when pressure is applied to or released from the piston 10.

Said axial play l1 of the O-ring 20 is defined to be in the range of d1*0.1 to d1*0.5. Consequently, the sum of the diameter d1 and the axial play l1 defines the length l2 of the sealing seat 18.

The O-ring 20 is formed as a circular sealing ring with a cord diameter d1 in its cross section that lies in the range of 0.7 to 2.5 mm, especially in the range of 1 to 2.2 mm, in particular 1.1 mm to 2.1. In particular said diameter d1 may be chosen to have a size of 1.2 mm, 1.78 mm or 2 mm. As can be seen in Fig. 1 for example, diameter d1 corresponds to the diameter of the cross section of the cord of the O-ring 20. That is, it defines how thick the O-ring 20 is.

In order to define the size of the O-ring 20 itself inner and outer ring diameters 32A and 32B are defined that lie in the range of 5 to 100 mm, in particular 8 to 40mm, for the inner diameter 32A, respectively 6 to 110 mm, in particular 8 to 44 mm for the outer diameter 32B.

Furthermore, the O-ring 20 is made out of an elastomeric material, such as thermoset materials, such as or thermoplastic materials. This allows the O-ring 20 to be compressed respectively decompressed upon application or release of a pressure.

A minimum O-ring compression at the front end 28 of the sealing seat 18 is selected in the range of 4 to 10%, especially 5 to 9% and most preferably of 7%. A maximum O-ring compression at the rear end 30 of the sealing seat 18 is selected in the range of 10 to 20%, especially 12 to 16% and most preferably of 14%.

In the following the functionality of the piston 10 according to the invention is described.

When the piston 10 is in its initial state, i.e. when no pressure is applied on it, the O-ring 20 can rest uncompressed and leaned against the top wall 38 in the sealing seat 18.

Once a user applies pressure on the piston 10 such that it is moved in a direction towards the front end 12 of the piston 10, the O-ring 20 starts to move in its groove 22. Because of accordingly chosen dimensions and materials of the O-ring 20 as well as the conically shaped outer wall 19 of the sealing seat 18 the O-ring 20 will first start to roll towards the back end 30 of the sealing seat 18.

That is, as long as the applied force is smaller than the friction force between the O-ring 20 and the outer wall 19 of the sealing seat 18, the O-ring 20 will roll over the outer surface 19 of the sealing seat 18. This ensures a smooth, even and linear response during extrusion as initial friction is eliminated and/or stick-slip effects can be avoided.

Once the applied force gets larger than the friction force the O-ring 20 continues sliding over the outer wall 19 of the sealing seat 18 towards the rear end 30 until it reaches the bottom wall 40 where it will be compressed such that the originally round cross section of the ring cord of the O-ring 20 will becomes rather oval.

Upon releasing of the axial force, the pre-load on the O-ring 20 is relieved which causes the piston 10 to retract slightly towards its default position at the front end 28 of the sealing seat 18. This retraction increases the internal volume in the plunger system respectively the cartridge such that a suck-back effect is created which prevents dripping of the material that is still near the outlet of the dispensing mechanism.

**Reference signs**

| | |
|---|---|
| piston | 10 |
| lower part | 11A |
| upper part | 11B |
| front end | 12 |
| rear end | 14 |
| outer wall | 16 |
| sealing seat | 18 |
| outer wall | 19 |
| O-ring | 20 |
| O-ring groove | 22 |
| inner wall | 24 |
| longitudinal axis | 26 |
| front end | 28 |
| rear end | 30 |
| inner ring diameter | 32A |
| outer ring diameter | 32B |
| front angle | 34 |
| rear angle | 36 |
| top wall | 38 |
| bottom wall | 40 |
| outer end | 42 |
| outer end | 44 |
| cartridge | C |
| depth at front | D1 |
| depth at back | D2 |
| angle | a1 |
| cord diameter | d1 |
| axial play | l1 |
| length | l2 |

## Claims

1. A piston (10) having a front end (12) and a rear end (14), with an outer wall (16) connecting the front end (12) to the rear end (14), wherein the outer wall (16) comprises a sealing seat (18) configured for receipt of an O-ring (20), and wherein the sealing seat (18) has a non-uniform cross-section in plane parallel to a longitudinal axis (26) of the piston (10), and with the cross-section at any axial height of the sealing seat (18) being circular in a plane perpendicular to the longitudinal axis (26) of the piston (10).

2. The piston according to claim 1, wherein the sealing seat (18) comprises a continuous outer surface.

3. The piston according to claim 1 or 2, wherein the sealing seat (18) is provided in the form of a conically shaped O-ring groove (22).

4. The piston according to one of claims 1 to 3, wherein the rear end (14) is an actuation end of the piston (10).

5. The piston according to one of claims 1 to 4, wherein a depth (D1) of the sealing seat (18) is larger towards the front end (12) of the piston (10) than a depth (D2) of the sealing seat (18) at the rear end (14) of the piston (10).

6. The piston according to one of claims 1 to 5, wherein an angle (a1) of an inner wall (24) of the sealing seat (18) with respect to the longitudinal axis (26) of the piston (10) that passes from the front end (12) to the rear end (14) is selected in the range of 5° to 20°.

7. The piston according to one of claims 1 to 6, wherein a minimum O-ring compression at a front end (28) of the sealing seat (18) is selected in the range of 4 to 10%, especially 5 to 9% and most preferably of 7%, and/or wherein a maximum O-ring compression at a rear end (30) of the sealing seat (18) is selected in the range of 10 to 20%, especially 12 to 16% and most preferably of 14%.

8. The piston according to one of claims 1 to 7, wherein a cross section of the O-ring (20) comprises a cord diameter (d1) in the range of 0.7 to 2.5 mm, especially in the range of 1 to 2.2 mm, in particular 1.1 mm to 2.1,
in particular wherein the cord diameter (d1) of the O-ring (20) has a size of 1.2 mm, 1.78 mm or 2 mm.

9. The piston according to one of claims 7 and 8, wherein a length (I2) between a front end (28) of the sealing seat (18) and a rear end (30) of the sealing seat (18) is the sum of the O-ring cord diameter (d1) and an axial play (11) of said O-ring (20), in particular with said axial play (11) of the O-ring (20) being in the range of d1*0.1 to d1*0.5.

10. The piston according to one of claims 3 to 9, wherein the conically shaped O-ring groove (22) of the sealing seat (18) comprises a rounded inner front angle (34), in particular with said inner front angle (34) comprising a radius (r1) in the range of d1*0.01 to d1*0.5.

11. The piston according to one of claims 3 to 10, wherein the conically shaped O-ring groove (22) of the sealing seat (18) comprises a rounded inner rear angle (36), in particular with said inner rear angle (36) comprising a radius (r2) in the range of d1*0.01 to d1*0.5.

12. The piston according to one of claims 1 to 11, in particular to one of claims 9 to 11, wherein the outer wall (16) of the piston (10) extends cylindrically from the rear end (14) to the sealing seat (18), in particular to the rear end (30) of the sealing seat (18).

13. The piston according to one of claims 1 to 12, in particular to one of claims 9 to 12, wherein the outer wall (16) of the piston (10) extends conically from the sealing seat (18) to the front end (12) of the piston (10), in particular from the rear end (30) of the sealing seat (18) to the front end (12) of the sealing seat (18).

14. The piston according to one of claims 1 to 13, wherein the sealing seat (18) comprises a top wall (38) and a bottom wall (40) extending transversely to the longitudinal axis (26), wherein the bottom wall (40) extends further from the longitudinal axis (26) than the top wall (38).

15. The piston according to one of claims 1 to 13, wherein the sealing seat (18) comprises a top wall (38) and a bottom wall (40) extending transversely to the longitudinal axis (26), wherein the bottom wall (40) extends the same distance from the longitudinal axis (26) as the top wall (38).

16. The piston according to one of claims 1 to 15, wherein the sealing seat (18) comprises a top wall (38) and a bottom wall (40) extending transversely to the longitudinal axis (26), wherein the top wall (38) and the bottom wall (40) extend parallel to one another.

17. The piston according to one of claims 14 to 16, wherein an outer end (42) of the top wall (38) is closer to the longitudinal axis (26) than an outer end (44) of the bottom wall (40).

18. The piston according to one of claims 1 to 17, wherein the sealing seat (18) comprises an outer diameter (D) in the range of 5 to 60 mm, with said diameter (D) reducing from the rear end (14) to the front end (12) of the piston (10).

19. The piston according to one of claims 1 to 18, wherein the O-ring (22) is made of an elastomeric material, in particular thermoset materials, such as or thermoplastic materials.

20. Cartridge optionally filled with a mastic material, the cartridge comprising a piston according to one of the preceding claims.
